# EUROPEAN PATENT APPLICATION

(11) **EP 4 636 066 A1**
(43) Date of publication of application: **22.10.2025**
(21) Application number: 23903076.0
(22) Date of filing: 03.10.2023
(51) Int. Cl.: C12M 1/00, C12Q 1/6844

(54) **NUCLEIC ACID AMPLIFICATION CHIP, NUCLEIC ACID AMPLIFICATION DEVICE, AND NUCLEIC ACID AMPLIFICATION METHOD**

(30) Priority: 16.12.2022 JP 2022201356
(71) Applicant: National Institute of Advanced Industrial Science and Technology, Chiyoda-ku Tokyo 100-8921 (JP)
(72) Inventor: NAGAI, Hidenori, Tsukuba-shi Ibaraki 305-8560 (JP); FURUTANI, Shunsuke, Tsukuba-shi Ibaraki 305-8560 (JP)
(74) Representative: Bandpay & Greuter
(86) International application number: PCT/JP2023/036095
(87) International publication number: WO 2024/127777

(57) **Abstract**

Provided is a nucleic acid amplification chip that enables high-speed movement of a solution more easily without measuring the movement of the solution by fluorescence detection, and suppresses the generation of air bubbles. The nucleic acid amplification chip is provided which includes a flow channel including, between a first connecting tube having a first opening and a second connecting tube having a second opening, a first protruding tube that is protruding in a first direction and communicates with the first connecting tube; a second protruding tube that is protruding in the first direction and communicates with the second connecting tube; and a tubular bridge section that allows fluid communication between the first protruding tube and the second protruding tube.

## Description

### Technical Field

The present invention relates to a nucleic acid amplification chip, a nucleic acid amplification device, and a nucleic acid amplification method.

### Background Art

Nucleic acid detection is a key technique for various fields such as pharmaceutical research and development, forensic science, clinical testing, and identification of agricultural crops and pathogenic microbial species. The ability to detect various diseases including cancer, microbial infections, genetic markers based on molecular phylogenetic analyses, and so on has become a general technique for disease and onset risk diagnosis, marker discovery, food and environmental safety assessment, criminal proof, and many other techniques.

The PCR method is a powerful technique for selectively amplifying a specific DNA segment. Using PCR, a target DNA sequence in template DNA can be copied to generate several millions of DNA fragment copies from the single template DNA. In PCR, through repetition of three or two phases of temperature conditions called a thermal cycle, individual reactions of denaturing DNA into single strands, annealing the denatured single-stranded DNA with primers, and extending the primers with a thermostable DNA polymerase enzyme are repeated in sequence.

Thus, the PCR method is the powerful method for exponentially amplifying genes through the thermal cycling. However, general-purpose thermal cycler devices used in PCR have a problem that slow temperature control due a huge thermal capacity of an aluminum block acting as a heater requires a long time for a PCR operation with 30 to 40 cycles. For this reason, there is a demand for speeding up of the PCR operation, and various methods have been developed to achieve this.

For example, various studies have been conducted on PCR operations on chips with microchannels formed therein, because it is believed that the reduction in a sample volume reduces the heat capacity and allows for faster thermal cycling. However, the use of a microchannel may result in insufficient heating or, conversely, overheating unless a desired amount of solution is transported accurately to a predetermined temperature area where a heater or the like is installed.

The present applicant proposed a nucleic acid amplification device which includes a liquid delivery mechanism using a microblower installed on a chip with a microchannel, and is capable of performing real-time PCR by detecting movement of a solution by fluorescence detection (Patent Literature 1).

### Citation List

### Patent Literature

PTL 1: Japanese Patent No. 6226284

### Summary of Invention

### Technical Problem

However, in some PCR methods, such as the Sanger method, only DNA amplification is required and a fluorescent probe for quantification is unnecessary. For this reason, it is not possible to detect movement of a solution by fluorescence detection. If one were to try to measure movement of a liquid using fluorescence detection, it would be necessary to either add an unnecessary fluorescent dye or use an additional detection method to confirm the speed of the liquid.

Furthermore, in a case where air bubbles are generated in a microchannel due to the air entrained in the microchannel along with a flow of a fluid into the microchannel, or due to the temperature during heating, stagnation occurs in the microchannel, hindering the movement of the solution.

In view of these problems, the present invention has an object to provide a nucleic acid amplification chip that enables high-speed movement of a solution more easily without measuring the movement of the solution by fluorescence detection, and suppresses the generation of air bubbles.

### Solution to Problem

In order to achieve the above object, a nucleic acid amplification chip of the present invention includes a flow channel including, between a first connecting tube having a first opening and a second connecting tube having a second opening, a first protruding tube that is protruding in a first direction and communicates with the first connecting tube; a second protruding tube that is protruding in the first direction and communicates with the second connecting tube; and a tubular bridge section that allows fluid communication between the first protruding tube and the second protruding tube.

Another embodiment of the present invention relates to a nucleic acid amplification device including the nucleic acid amplification chip according to an embodiment of the present invention, a first heater, a second heater, and a gas transport device.

Still another embodiment of the present invention relates to a nucleic acid amplification method using the nucleic acid amplification device according to an embodiment of the present invention.

### Advantageous Effects of Invention

According to the present invention, it is possible to provide a nucleic acid amplification chip that enables high-speed movement of a solution more easily without measuring the solution movement by fluorescence detection, and suppresses the generation of air bubbles.

### Brief Description of Drawings

[Fig. 1] Fig. 1 is a schematic view of a nucleic acid amplification device according to an embodiment of the present invention.
[Fig. 2] Fig. 2 is a modification of a nucleic acid amplification chip of the present invention.
[Fig. 3] Fig. 3 is a modification of a nucleic acid amplification chip of the present invention.
[Fig. 4] Fig. 4 is a modification of a nucleic acid amplification chip of the present invention.
[Fig. 5] Fig. 5 is a modification of a nucleic acid amplification chip of the present invention.

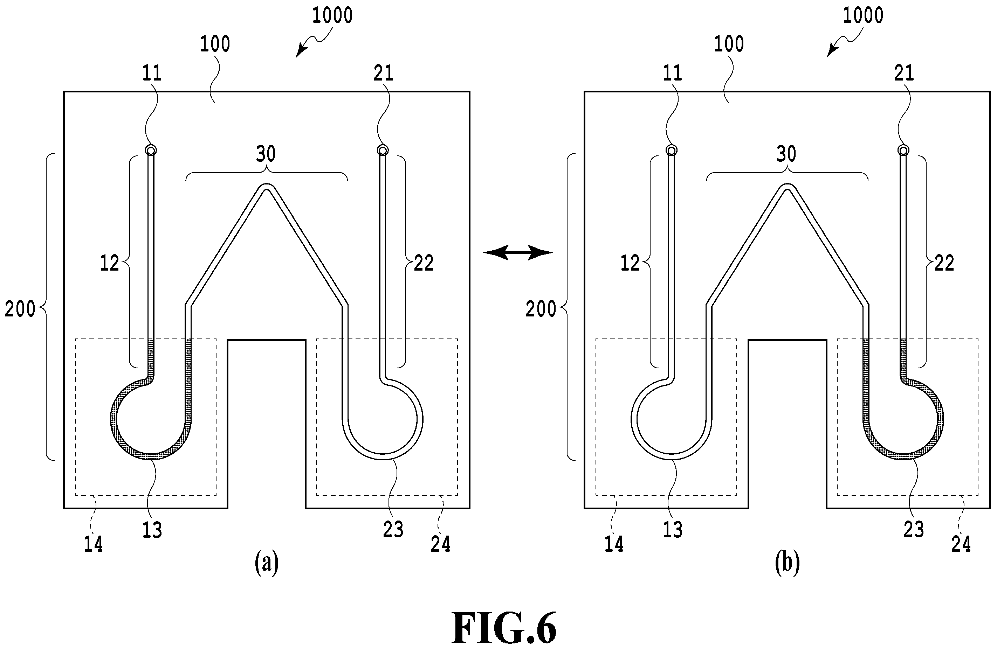

[Fig. 6] Fig. 6 includes diagrams presenting nucleic acid amplification performed using a nucleic acid amplification device according to an embodiment of the present invention.

### Description of Embodiments

Hereinafter, embodiments of the present invention will be described in detail. However, the present invention should not be limited to these embodiments.

### (Nucleic Acid Amplification Device)

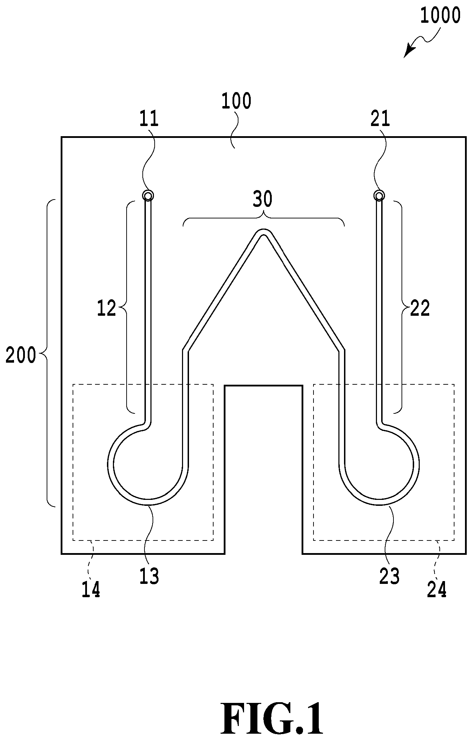

Fig. 1 is a schematic view of a nucleic acid amplification device 1000 according to an embodiment of the present invention. As illustrated in Fig. 1, the nucleic acid amplification device 1000 includes a nucleic acid amplification chip 100, a first heater 14, a second heater 24, and a gas transport device (not illustrated). The nucleic acid amplification chip 100 includes a flow channel 200 including a first connecting tube 12 having a first opening 11, a first protruding tube 13 protruding in a first direction (also simply referred to as the "first protruding tube"), a second connecting tube 22 having a second opening 21, a second protruding tube 23 protruding in the first direction (also simply referred to as the "second protruding tube"), and a bridge section 30. In the nucleic acid amplification device 1000, the first direction of the nucleic acid amplification chip 100 is aligned with the gravity direction. Hereinafter, each of components will be described in detail with reference to the nucleic acid amplification device 1000 in Fig. 1.

### (Nucleic Acid Amplification Chip)

The nucleic acid amplification chip 100 is a chip that retains a PCR solution containing a gene desired to be amplified in the flow channel 200 and exponentially amplifies the gene by repeating thermal cycling.

The nucleic acid amplification chip 100 includes the flow channel 200 including the first connecting tube 12 having the first opening 11, the first protruding tube 13 protruding in the first direction, the second connecting tube 22 having the second opening 21, the second protruding tube 23 protruding in the first direction, and the bridge section 30.

The first connecting tube 12 has the first opening 11 and communicates with an end of the first protruding tube 13.

The first opening 11 is provided to the first connecting tube 12 and makes the flow channel 200 open to the outside of the nucleic acid amplification chip 100. From the first opening 11, a gas or a liquid such as a PCR solution can be injected into the flow channel 200 by using a gas transport device to be described later, a dispenser, or the like. Conversely, the gas or the liquid in the flow channel 200 can be discharged from the first opening 11.

A connection angle R₁ between the first connecting tube 12 and the first protruding tube 13 is preferably 90° or larger. Moreover, the first connecting tube 12 preferably extends in parallel to the first direction. With this structure, in a case where the nucleic acid amplification chip 100 is set in the nucleic acid amplification device 1000 with the first direction aligned with the gravity direction, a liquid injected into the first connecting tube 12 flows into the first protruding tube 13 along the gravity direction. In addition, if the gas is present in the flow channel 200, air bubbles reach the first opening 11 through the first connecting tube 12 and then are discharged.

The first protruding tube 13 protruding in the first direction is a section that retains the PCR solution and is heated to a first temperature range by the first heater 14 to be described later, thereby causing a first PCR reaction.

The first protruding tube 13 is formed so as to have a portion protruding in the first direction (protruding section). The PCR solution injected from the first connecting tube 12 can be retained in the protruding section. The protruding section may be, for example, in a U shape or a V shape.

Since the PCR solution is retained and heated to cause the reaction in the first protruding tube 13, it is preferable that the size of the first protruding tube 13 be large enough to retain a solution in an amount required to carry out a desired PCR reaction.

The second opening 21, the second connecting tube 22, and the second protruding tube 23 are the same as the first opening 11, the first connecting tube 12, and the first protruding tube 13, respectively, except that the temperature for a PCR reaction to be caused in the second protruding tube 23 is different from the first temperature range. The second opening 21, the second connecting tube 22, and the second protruding tube 23 may have shapes symmetric to or different from the first opening 11, the first connecting tube 12, and the first protruding tube 13, respectively. As will be described later, the PCR solution is repeatedly transported between the first protruding tube 13 and the second protruding tube 23 alternately. To this end, if the first direction is aligned with the gravity direction, the heights of the first protruding tube 13 and the second protruding tube 23 in the gravity direction are preferably equal to each other.

The bridge section 30 is tubular and allows fluid communication between the first protruding tube 13 and the second protruding tube 23. The bridge section 30 is arranged away from the first protruding tube and the second protruding tube in the direction opposite to the first direction. In the case where the nucleic acid amplification chip 100 is set with the first direction aligned with the gravity direction, the bridge section 30 sandwiched between the adjacent first protruding tube 13 and second protruding tube 23 is positioned at a higher height in the gravity direction than these two protruding tubes are. For this reason, once the PCR solution transported from one of the protruding tubes moves beyond the bridge section 30, the PCR solution can flow into the other protruding tube according to the gravity.

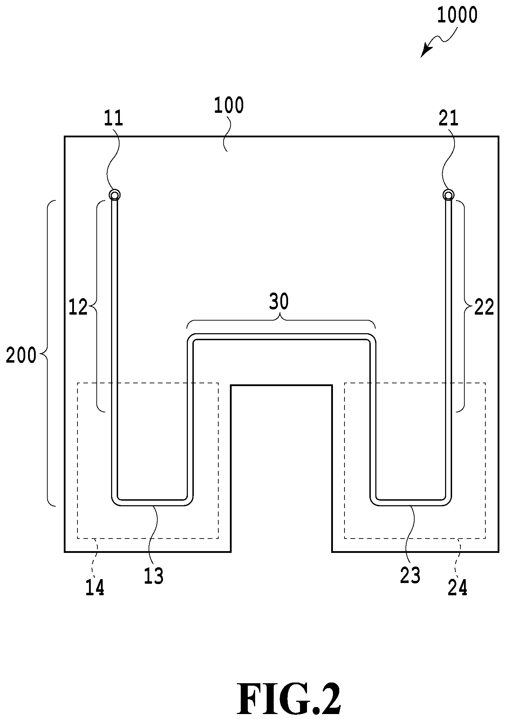

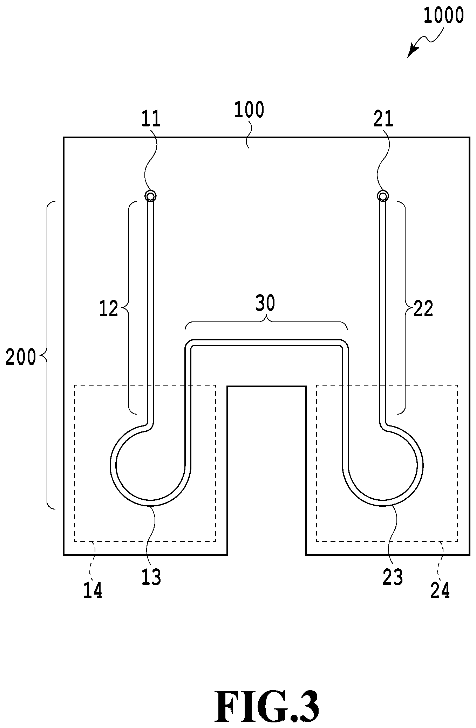

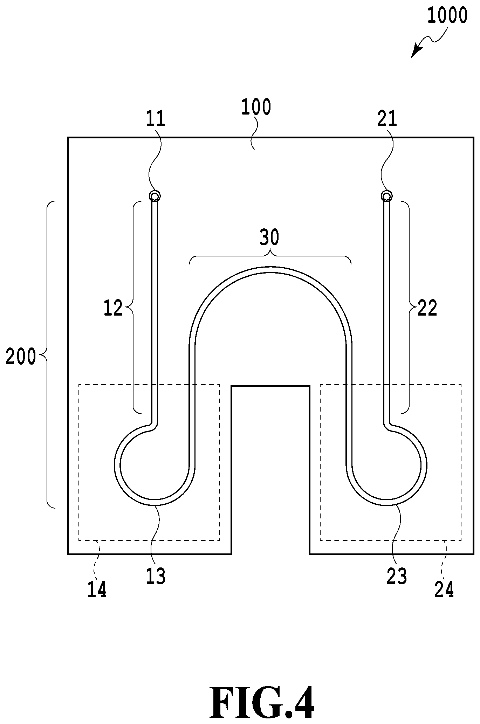

The bridge section 30 may have a bridge shape extending in the direction perpendicular to the first direction (Figs. 2 and 3) or a shape projecting in the direction opposite to the first direction (Figs. 1 and 4). For example, the bridge section 30 may have a mountainous shape protruding in the direction opposite to the first direction (Fig. 1) or a semi-circular shape protruding in the direction opposite to the first direction (Fig. 4). It is preferable that the bridge section 30 be in a shape protruding in the direction opposite to the first direction, because, once the solution is transported to the midpoint of the bridge section 30 (the infection point of the protruding portion), the solution can be automatically transported into the other protruding tube.

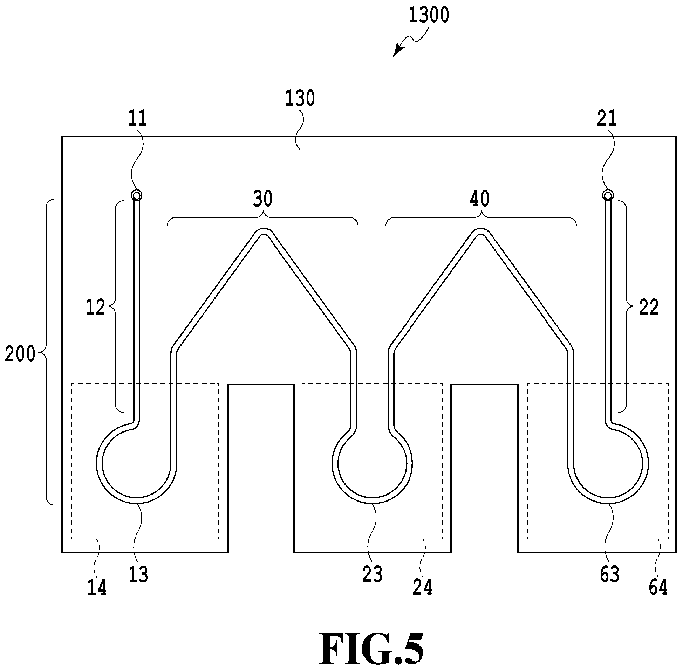

Alternatively, three or more protruding tubes may be provided between the first connecting tube 12 and the second connecting tube 22, as long as the shape is such that a bridge section is provided between each pair of adjacent protruding tubes. In Fig. 5, a first protruding tube 13, a bridge section 30, a second protruding tube 23, a second bridge section 40, and a third protruding tube 63 are provided in this order between a first connecting tube 12 and a second connecting tube 22. The adjacent protruding tubes are heated to temperature ranges different from each other, and allow respective PCR reactions to occur. The non-adjacent protruding tubes, for example, the first protruding tube 13 and the third protruding tube 63, may be heated to different temperature ranges or the same temperature range.

Connection angles of the bridge section 30 with the first protruding tube 13 and the second protruding tube 23 and connection angles of the second bridge section 40 with the second protruding tube 23 and the third protruding tube 63 are preferably 90° or more. With such connection angels, air bubbles can be removed from the solution without being trapped in these connection portions.

The flow channel 200 in the nucleic acid amplification chip 100 is preferably formed on a plane parallel to the first direction. Thus, in the case where the first direction is aligned with the gravity direction, the solution in the flow channel 200 can move according to the gravity.

The flow channel 200 in the nucleic acid amplification chip 100 may be in a cylindrical shape in which the shape of an inner cavity cross section taken perpendicularly to the center axis (hereinafter, the shape will be simply referred to as a "cross-sectional shape") is a circular or elliptical shape or may be a tube with a polygonal cross-sectional shape. The flow channel 200 may be in a shape in which the cross-sectional shape is composed of a combination of curved portions and polygonal portions. The flow channel 200 preferably has a cross-sectional shape uniform throughout the flow channel.

The flow channel 200 is preferably a microchannel. A maximum value of a length connecting two points on an outer circumference of the cross-sectional shape of the flow channel 200 is preferably 0.5 mm or more. For example, in a case where the cross-sectional shape is a circular shape, the diameter is preferably 0.5 mm or more. Instead, in a case where the cross-sectional shape is an elliptical shape, the radius of the major axis is preferably 0.25 mm or more. In a case where the cross-sectional shape is a polygonal shape, the maximum value of the length of a diagonal line is preferably 0.5 mm or more. Having such size, the flow channel 200 can allow the solution to move to a desired position according to the gravity, because the gravity effect exceeds a frictional force between the solution and the inner wall of the flow channel. The speed of movement in the gravity direction in the flow channel 200 is preferably 5 mm/s or more.

Moreover, the flow channel 200 is preferably in a microchannel size in which the solution can be maintained in a plug-flow form. The maximum value of the length connecting the two points on the outer circumference of the cross-sectional shape of the flow channel 200 is preferably 1 mm or less. For example, in the case where the cross-sectional shape is the circular shape, the diameter is preferably 1 mm or less. Instead, in the case where the cross-sectional shape is the elliptical shape, the radius of the major axis is preferably 0.5 mm or less. In the case where the cross-sectional shape is the polygonal shape, the maximum value of the length of the diagonal line is preferably 1 mm or less.

A material used for the nucleic acid amplification chip 100 is preferably a material that has relatively high thermal conductivity, is stable in the temperature ranges required for PCR, is resistant to corrosion by electrolyte solutions and organic solvents, and does not adsorb nucleic acids or proteins. For example, glass, quartz, silicon, resin, and metal may be used as the material. The preferred material is a resin such as polymethyl methacrylate (PMMA) or cycloolefin polymer (COP).

The nucleic acid amplification chip 100 can be formed by injection molding or MEMS process.

### (Heater)

The heater heats the solution in the corresponding protruding tube to a specific temperature range. The nucleic acid amplification device 1000 in Fig. 1 includes the first heater 14 and the second heater 24.

The first heater 14 is a heater to heat the solution in the first protruding tube 13 to a first temperature range. To heat the first protruding tube 13, the first heater 14 is installed so as to cover the back side of the first protruding tube 13 of the nucleic acid amplification chip 100.

The second heater 24 is a heater to heat the solution in the second protruding tube 23 to a second temperature range that is different from the first temperature range. To heat the second protruding tube 23, the second heater 24 is installed so as to cover the back side of the second protruding tube 23 of the nucleic acid amplification chip 100.

The first temperature and the second temperature are determined depending on the thermal cycle of the PCR reactions (a denaturation temperature range and an extension/annealing temperature range), respectively. In the case where three or more heaters are used as in Fig. 5, different temperature ranges are set for the adjacent heaters. The non- adjacent heaters, for example, the first heater 14 and a third heater 64, may heat the solution to different temperature ranges or the same temperature range.

### (Gas Transport Device)

The nucleic acid amplification device 1000 includes at least one gas transport device. The gas transport device is connected to at least one of the first opening 11 and the second opening 21, and injects gas into the flow channel 200 or discharges gas from the flow channel 200. By injecting or discharging the gas, the gas transport device can move the PCR solution in the flow channel 200.

The gas transport device may be any device capable of injecting or discharging a desired volume of gas into or from the flow channel 200, and the pressure in the flow channel 200 is released while the gas transport device is stopped. For example, a microdispenser or a microblower may be used as the gas transport device. For example, a microdosing Unit Type-7615 manufactured by Burkert may be used as the microdispenser, and a microblower MZB 100T02 manufactured by Murata Manufacturing Co., Ltd. may be used as the microblower.

The gas transport device or devices may be connected to any one or both of the first opening 11 and the second opening 21. In the case where the gas transport devices are connected to both of the openings, the gas transport devices are alternately operated depending on a direction of liquid transport. In the case where the gas transport devices are connected to both of the openings, the pressure in the flow channel 200 is also released while the gas transport devices are stopped.

In the case where the gas transport device is connected to any one of the openings, the gas transport device is capable of moving the solution by injecting the gas into the flow channel 200 and moving the solution by sucking the gas from the flow channel 200. In any case, while the gas transport device or devices are stopped, the pressure is released and the solution moves in the flow channel 200 according to the gravity.

### (Nucleic Acid Amplification Method)

Next, a nucleic acid amplification method using the nucleic acid amplification device 1000 will be described. Although the following description will be given using an embodiment in which the gas transport device is provided to the first opening 11, the method is not limited to this case.
(1) The nucleic acid amplification device 1000 is installed so that the first direction of the nucleic acid amplification chip 100 is aligned with the gravity direction.
(2) By use of a micropipette or the like, 5 to 25 µL of a PCR solution is measured and injected into the flow channel 200 from the first opening 11. The PCR solution flows into the first protruding tube 13 through the first connecting tube 12 and is retained in the first protruding tube 13 (Fig. 5(a)). The PCR solution only have to be capable of amplifying gene sequences and does not need to contain any fluorescent dye. Instead, the PCR solution may contain a fluorescent dye. As an example of a PCR reagent, a PCR premixture may be used which is prepared by using SpeedSTAR (registered trademark) HS DNA polymerase, manufactured by Takara Bio Inc., at a final concentration of 0.025 U/µL, mixing it with the ancillary FAST Buffer I and dNTP Mixture at the concentrations specified in the manual, and then mixing the resultant with 0.5 µM primers. The composition of the PCR reagent is not limited to this, but may be any reagent composition for PCR.
(3) The first heater 14 heats the PCR solution in the first protruding tube 13 to the denaturation temperature (about 95°C) to dissociate double-stranded DNA into single strands.
(4) After the reaction is completed, the gas transport device injects gas to transport the PCR solution so that the solution can move beyond the bridge section 30, and then is stopped. With release of the pressure in the flow channel 200, the solution having moved beyond the bridge section 30 moves into the second protruding tube 23 according to the gravity and is retained in the second protruding tube 23 (Fig. 5(b)).
(5) The second heater 24 heats the PCR solution in the second protruding tube 23 to the extension/annealing temperature (50 to 70°C) to bind the primers and replicate a region between the two types of primer sequences from the single-stranded template DNA, thereby generating the double-stranded DNA.
(6) The gas transport device sucks the gas in the flow channel 200 to transport the PCR solution so that the solution can move from the second protruding tube 23 beyond the bridge section 30, and then is stopped. As in the forward path, with release of the pressure in the flow channel 200, the solution having moved beyond the bridge section 30 moves into the first protruding tube 13 according to the gravity and is retained in the first protruding tube 13.

The operations (3) to (6) are repeated to change the temperature, so that the target DNA sequences can be exponentially amplified.

In the present embodiment, once the PCR solution moves beyond the bridge section 30, the PCR solution flows according to the gravity into the protruding tube to which the solution is to be transported, and can be retained therein as described above. Therefore, strict adjustment for the solution transport is unnecessary. According to the present embodiment, the gas is repeatedly injected into and sucked from the flow channel 200 to the extent that the solution moves beyond the bridge section 30, which makes it possible to transport the solution to each of the desired positions, thereby enabling the thermal cycling.

In the present embodiment, since the first direction of the nucleic acid amplification chip 100 is aligned with the gravity direction, generated air bubbles move in the direction opposite to the first direction, consequently move to the first opening 11 or the second opening 21 or the bridge section 30, and then can be removed from the flow channel 200. The removal of the air bubbles makes it possible to avoid accidental errors in the thermal cycling.

Although the various embodiments and modifications are described above, the present invention should not be limited to the description of these. Other embodiments conceivable within the scope of the technical idea of the present invention are also included in the scope of the present invention.

Moreover, the above embodiments and modifications may be each carried out alone or two or more of them may be combined as appropriate.

### Reference Signs List

- 11: first opening
- 12: first connecting tube
- 13: first protruding tube
- 14: first heater
- 21: second opening
- 22: second connecting tube
- 23: second protruding tube
- 24: second heater
- 30: bridge section
- 100: nucleic acid amplification chip
- 200: flow channel

## Claims

1. A nucleic acid amplification chip comprising a flow channel including:
between a first connecting tube having a first opening and a second connecting tube having a second opening,
a first protruding tube that is protruding in a first direction and communicates with the first connecting tube;
a second protruding tube that is protruding in the first direction and communicates with the second connecting tube; and
a tubular bridge section that allows fluid communication between the first protruding tube and the second protruding tube.

2. The nucleic acid amplification chip according to claim 1, wherein the bridge section is arranged away from the first protruding tube and the second protruding tube in a direction opposite to the first direction.

3. The nucleic acid amplification chip according to claim 1, wherein the first connecting tube, the first protruding tube, the second connecting tube, the second protruding tube, and the bridge section have the same shape in inner cavity cross sections taken perpendicularly to a center axis of the flow channel.

4. The nucleic acid amplification chip according to claim 1, wherein a connection angle between each pair of the tubes communicating with each other is 90° or larger.

5. The nucleic acid amplification chip according to claim 3, wherein a maximum value of a length connecting two points on an outer circumference of the inner cavity cross section of the flow channel is 0.5 mm or more.

6. The nucleic acid amplification chip according to claim 5, wherein the above maximum value of the flow channel is 1 mm or less.

7. The nucleic acid amplification chip according to claim 1, wherein the bridge section is protruding in a direction opposite to the first direction.

8. The nucleic acid amplification chip according to claim 1, which is installed in a nucleic acid amplification device, wherein
the first direction is a gravity direction, and
a speed of movement in the first direction in the flow channel is 5 mm/s or more.

9. The nucleic acid amplification chip according to claim 1, further comprising a third protruding tube and a second bridge section.

10. A nucleic acid amplification device comprising:
the nucleic acid amplification chip according to any one of claims 1 to 9;
a first heater;
a second heater; and
a gas transport device, wherein
the first heater heats the first protruding tube at a first temperature,
the second heater heats the second protruding tube at a second temperature that is different from the first temperature, and
the gas transport device transports gas in the flow channel.

11. The nucleic acid amplification device according to claim 10, wherein the gas transport device is a microdispenser or a microblower.

12. The nucleic acid amplification device according to claim 10, wherein the first direction of the nucleic acid amplification chip is a gravity direction.

13. A nucleic acid amplification device comprising:
the nucleic acid amplification chip according to claim 9;
a first heater;
a second heater;
a third heater; and
a gas transport device, wherein
the first heater heats the first protruding tube at a first temperature,
the second heater heats the second protruding tube at a second temperature that is different from the first temperature,
the third heater heats the third protruding tube at a third temperature, and
the gas transport device transports gas in the flow channel.

14. A nucleic acid amplification method comprising the steps of:
installing, in the nucleic acid amplification device according to claim 10, the nucleic acid amplification chip such that the first direction is aligned with a gravity direction;
injecting a nucleic acid amplification solution from the first opening into the first protruding tube;
causing the first heater to heat the nucleic acid amplification solution in the first protruding tube to a first temperature;
causing the gas transport device to transport gas, thereby transporting the nucleic acid amplification solution to a position beyond the bridge section, and causing the nucleic acid amplification solution to flow into the second protruding tube; and
causing the second heater to heat the nucleic acid amplification solution in the second protruding tube to a second temperature.

15. The nucleic acid amplification method according to claim 14, further comprising the step of causing the gas transport device to transport gas, thereby transporting the nucleic acid amplification solution to a position beyond the bridge section, and causing the nucleic acid amplification solution to flow into the first protruding tube.
